# EUROPEAN PATENT APPLICATION

(11) **EP 0 600 619 A1**
(43) Date of publication of application: **08.06.1994**
(21) Application number: 93308887.4
(22) Date of filing: 08.11.1993
(51) Int. Cl.: A61N 5/06

(54) **Tanning apparatus**

(30) Priority: 07.11.1992 GB 9223387
(71) Applicant: Turnley, David James, Reigate, Surrey RH2 8JU (NL)
(72) Inventor: Turnley, David James, Reigate, Surrey RH2 8JU (NL)
(74) Representative: Skinner, Michael Paul

(57) **Abstract**

A tanning booth (10) comprising a compartment (12) and tanning means (14) located in the wall (22) of the compartment (12). The compartment (12) comprises an opening (28) and a curtain (40) movable across the opening (28). A sensor is provided to sense when the curtain is closed over the opening (28), and to prevent operation of the tanning means until the curtain (40) is properly closed. A lock (50) enables the curtain to be locked closed. A stool (38) is provided in the compartment, and with a thermostatically controlled fan to ventilate the compartment (12). Reflective panels (30) are provided inside the compartment (12) to reflect radiation from the tanning means (14) around the compartment (12). The booth (10) is coin and/or token operated, and a timer is provided to allow the user to select the amount of time of operation of the tanning means (14) in accordance with the value of coins or tokens inserted. A goggle dispenser (44) is provided.

## Description

The present invention relates to tanning apparatus. The term "tanning" is used herein to refer to the effect of modifying the colouration of human skin by exposure to light of various wavelengths, particularly ultra-violet light.

According to the present invention there is provided tanning apparatus comprising a compartment to accommodate a user, and tanning means operable to produce a tanning effect on a user.

Preferably the compartment comprises an entrance which may be closed to render the compartment substantially light-proof. Closure means such as a curtain, door or the like may be provided for the entrance. The closure means may comprise lock means. The lock means may be operable to lock while the tanning means is in operation. Alternatively or additionally, the lock means may be manually operable from inside the compartment.

Sensor means may be provided to sense closure of the closure means and prevent the tanning means operating unless closure is sensed.

Preferably the compartment further comprises reflective means on the inside thereof to reflect tanning radiation from the tanning means. The reflective means may comprise reflective panels on one or more inner surfaces of the compartment.

Preferably ventilation means is provided to ventilate the compartment. A fan may be provided, which may be thermostatically controlled.

A seat may be provided in the compartment. Preferably the seat is adjustable, preferably height adjustable, to enable the user to adjust its position relative to the tanning means. Reference means may be provided to assist the user in adopting a suitable position relative to the tanning means.

Preferably the tanning means is located on or in a wall of the compartment. The tanning means may comprise one or more tanning tubes, such as ultra violet radiation emitting tubes.

A shield may be provided over the tanning means to protect the said tanning means, whilst permitting radiation to pass therethrough.

The tanning means may be so arranged as to allow irradiation of at least the face of a user.

The apparatus may also comprise safety goggle dispensing means.

The apparatus may comprise actuating means to actuate operation of the tanning means. The actuating means may be operable upon insertion of a token or coin into the apparatus.

Preferably timing means is provided to set the period of time for which the tanning means operates. The timing means may be adjustable, for instance according to the value of coins/tokens inserted in the apparatus.

Preferably the apparatus is portable, and may be self-contained. The apparatus is preferably a unitary structure.

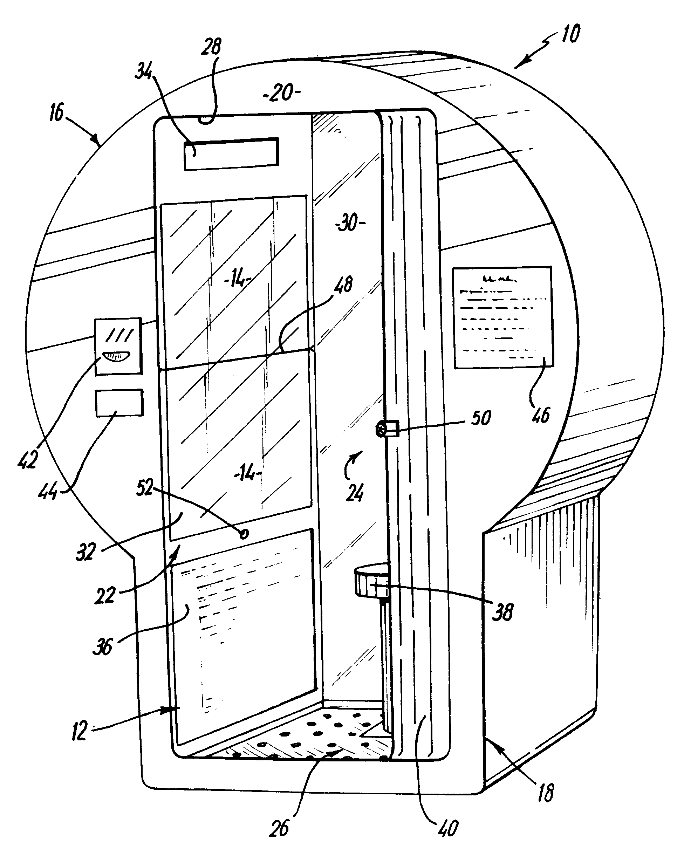

An embodiment of the present invention will now be described by way of example only, with reference to the accompanying drawing which is a perspective view of one embodiment of the invention.

The drawing shows a tanning booth 10 which comprises a compartment shown generally at 12, and tanning means indicated generally at 14. The outer form of the booth 10 has an upper portion 16 with a circular outline and a lower portion 18 of rectangular outline. The front 20 and rear of the booth 10 are generally flat, giving the booth 10 a generally rectangular outline when viewed from the side.

The compartment 12 is defined within the booth 10 by three substantially planar walls (two of which are shown at 22, 24), a ceiling panel (not shown), a floor panel 26 and an opening 28.

The tanning means 14 is located in a cavity between the wall 22 and the external circular surface 16. The tanning means comprises a plurality of conventional ultra violet tanning tubes (not shown), covered by a shield 32 which is transparent to ultra-violet light. The shield protects the tubes from damage or vandalism for instance, whilst allowing radiation to pass therethrough. The shield 32 extends approximately two thirds of the way down the wall 22.

A duct 34 above the shield 32 leads to a fan (not shown). The fan is located in the cavity behind the shield 32 and is thermostatically controlled to ventilate the booth 10 and maintain a constant temperature therein.

Beneath the shield 32 is an instruction and information panel 36 for the user to refer to when in the booth 10.

A reflective panel 30 extends over the wall 24 to reflect radiation emitted from the tanning means 14. A reflective panel may also be provided on other surfaces of the compartment.

A stool 38 is provided in the booth for the user, and has an adjustable height.

A curtain 40 is provided with which the opening 28 can be completely closed, to be light tight. A sensor is also provided to sense when the curtain is closed over the opening 28, and to prevent operation of the tanning tubes unless the curtain 40 is properly closed. A lock 50 enables the curtain to be locked closed. The sensor may be incorporated in the lock.

The booth 10 is coin and/or token operated and a coin-feed mechanism is provided behind a panel 42 on the front 18 of the booth 10 for insertion of coins and/or tokens.

A timer (not shown) is provided, perhaps as part of the panel 42, to allow the user to select the amount of time he/she wishes the tanning tubes to operate. This selection is made by depositing the appropriate number/ value of coins or tokens. It may, for instance be possible to select periods of five, ten or fifteen minutes, but other durations could be made available.

A goggle dispenser 44 is also provided to dispense a pair of safety goggles to the user. The goggles are automatically dispensed when the correct value of coins/ tokens has been inserted.

A further instruction and description panel 46 is fixed to the front 18 of the booth 10.

In use, a user first decides the desired length of time for which the tanning tubes are to operate. He/she then inserts the required value of coin(s)/token(s) through the panel 42. A pair of safety goggles will then be dispensed. It is to be appreciated that the length of time of operation may be automatically determined by the value of coins or tokens inserted, and separate manual selection is not required.

The user then enters the booth 10 and may adjust the height of the stool 38 as desired. A reference line 48 on the shield 32 helps the user to adopt a favourable tanning position relative to the tubes.

Once seated as desired, the user closes the curtain 40, ensuring it is fully closed. This is sensed by the sensor to ensure that the compartment is light tight. The user may lock the door using the lock 50. If desired, the user may then remove articles of clothing, for instance hats, jackets, etc. In an alternative, the door may lock and unlock automatically with the operation of the tanning tubes.

When ready, the user switches the tubes on using the start button 52 beneath the shield 32.

The tubes then operate for the selected period of time. The fan 34 ensures that the temperature within the booth is retained at a comfortable level for the user. The reflective panels 30 facilitate production of an even tan.

Once the time has elapsed, the tubes and fan are automatically switched off. The user can then leave the booth 10.

It is to be appreciated that the booth is a self-contained unit, which requires only an electrical supply in order to operate. It can be located in public places, such as public transport stations, holiday resorts and the like.

Various modifications may be made without departing from the spirit and scope of the present invention. For example, the booth may be of any shape. The modes of operation of the sensor, fan, lock, goggle dispenser and tanning tubes may be varied. Tanning tubes may be provided in more than one wall, and may be of any type.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. Tanning apparatus (10) characterised by comprising a compartment (12) to accommodate a user, and tanning means (14) operable to produce a tanning effect on a user.

2. Tanning apparatus according to claim 1, characterised in that the compartment (12) comprises an entrance (28) which may be closed to render the compartment (12) substantially light-proof.

3. Tanning apparatus according to claim 2, characterised in that the compartment is closed by closure means (40) such as a door or curtain.

4. Tanning apparatus according to claim 3, characterised in that the closure means (40) comprises lock means (50).

5. Tanning apparatus according to claim 4, characterised in that the lock means (50) is manually operable from inside the compartment (12).

6. Tanning apparatus according to any of claims 3 to 5, characterised in that sensor means is provided to sense closure of the closure means (40) and prevent the tanning means (14) operating unless closure is sensed.

7. Tanning apparatus according to any preceding claim, characterised in that the compartment (12) further comprises reflective means (30) on the inside thereof to reflect tanning radiation from the tanning means (14).

8. Tanning apparatus according to any preceding claim, characterised in that ventilation means (34) is provided to ventilate the compartment (12).

9. Tanning apparatus according to any preceding claim, characterised in that an automatically operable fan is provided.

10. Tanning apparatus according to any preceding claim, characterised in that a seat (38) is provided in the compartment (12).

11. Tanning apparatus according to claim 10, characterised in that the seat (38) is adjustable to enable the user to adjust its position relative to the tanning means (14).

12. Tanning apparatus according to any preceding claim, characterised in that reference means is provided to assist the user in adopting a suitable position relative to the tanning means (14).

13. Tanning apparatus according to any preceding claim, characterised in that the tanning means (14) comprises one or more tanning tubes, such as ultra violet radiation emitting tubes.

14. Tanning apparatus according to any preceding claim, characterised in that a shield (32) is provided over the tanning means (14) to protect the said tanning means (14), whilst permitting radiation to pass therethrough.

15. Tanning apparatus according to any preceding claim, characterised in that the tanning means (14) is so arranged as to allow irradiation of at least the face of a user.

16. Tanning apparatus according to any preceding claim, characterised in that the apparatus comprises safety goggle dispensing means (44).

17. Tanning apparatus according to any preceding claim, characterised in that the apparatus (10) comprises actuating means (52) operable to actuate operation of the tanning means upon insertion of a token or coin into the apparatus.

18. Tanning apparatus according to any preceding claim, characterised in that timing means is provided to set the period of time for which the tanning means operates.

19. Tanning apparatus according to claim 18, characterised in that the timing means is adjusted according to the value of coins/tokens inserted in the apparatus.

20. Tanning apparatus according to any preceding claim, characterised in that the apparatus (10) is portable.
